# EUROPÄISCHE PATENTANMELDUNG

(11) **EP 4 796 076 A1**
(43) Veröffentlichungstag der Anmeldung: **26.08.2026**
(21) Anmeldenummer: 26152786.5
(22) Anmeldetag: 20.01.2026
(51) Int. Cl.: A61C 19/00, A61B 90/70, A61L 2/18

(54) **ADAPTIONSLEISTE FÜR EIN REINIGUNGSGERÄT UND REINIGUNGSGERÄT**

(30) Priorität: 20.02.2025 BE 202500021
(71) Anmelder: Miele & Cie. KG, 33332 Gütersloh (DE)
(72) Erfinder: Goldberg, Gregor, 48231 Warendorf (DE)

(57) **Zusammenfassung**

Die Erfindung betrifft eine Adaptionsleiste (AL) für ein Reinigungsgerät zur Aufnahme mindestens eines medizinischen Instruments, insbesondere für ein Dentalpflegegerät, wobei die Adaptionsleiste (AL) zumindest ein Aufnahmeelement (AS) für einen Steckadapter, welches dazu ausgeformt ist, um das medizinisches Instrument aufzunehmen, zumindest eine Medienleitung (400) die in der Adaptionsleiste (AL) ausgebildet ist, welche mit einem Versorgungskanal (KS) verbunden ist, um ein Reinigungsmedium zu dem medizinischen Instrument zu fördern oder zu leiten, und ein Adaptersperrelement (ADS) aufweist, wobei das Adaptersperrelement (ADS) beweglich ausgeformt ist, um das Aufnahmeelement (AS) zum Aufstecken des Steckadapter des medizinischen Instruments freizugeben, wenn das Adaptersperrelement (ADS) in einer ersten Stellung ist und/oder das Aufnahmeelement (AS) zu sperren, wenn das Adaptersperrelement (ADS) in einer zweiten Stellung ist, wobei das Adaptersperrelement (ADS) zumindest ein Sperrstück (SF) zum Sperren eines Durchflusses des Reinigungsmediums von dem Versorgungskanal (KS) die Medienleitung (400) aufweist, das derart ausgebildet ist um in der ersten Stellung außerhalb der Medienleitung (400) angeordnet zu sein, und in der zweiten Stellung innerhalb der Medienleitung (400) angeordnet ist.

## Beschreibung

Die Erfindung betrifft eine Adaptionsleiste für ein Reinigungsgerät und ein Reinigungsgerät mit einer Adaptionsleiste gemäß den Hauptansprüchen.

Die EP 0638297 B1 beschreibt ein Verfahren und eine Vorrichtung zur hygienischen Aufbereitung von medizinischen, insbesondere zahnmedizinischen Instrumenten mit oder ohne Medienkanälen sowie mit oder ohne beweglichen Innenteilen.

Der hier vorgestellte Ansatz stellt sich die Aufgabe, eine verbesserte Adaptionsleiste für ein Reinigungsgerät und ein verbessertes Reinigungsgerät zu schaffen.

Erfindungsgemäß wird diese Aufgabe durch eine Adaptionsleiste für ein Reinigungsgerät und ein Reinigungsgerät mit den Merkmalen der Hauptansprüche gelöst. Vorteilhafte Ausgestaltungen und Weiterbildungen der Erfindung ergeben sich aus den nachfolgenden Unteransprüchen.

Die mit der Erfindung erreichbaren Vorteile bestehen neben einer kontrollierten Ausgabe von Reinigungsmittel und/oder Pflegemittel aus einer Reduzierung des Einsatzes von nicht benötigtem Pflegeöl aus nicht bestückten Adaptern.

Es wird eine Adaptionsleiste für ein Reinigungsgerät zur Aufnahme mindestens eines medizinischen Instruments, insbesondere für ein Dentalpflegegerät vorgestellt, wobei die Adaptionsleiste zumindest ein Aufnahmeelement für einen Steckadapter, welches dazu ausgeformt ist, um das medizinisches Instrument aufzunehmen, zumindest eine Medienleitung die in der Adaptionsleiste ausgebildet ist, welche mit einem Versorgungskanal verbunden ist, um ein Reinigungsmedium zu dem medizinischen Instrument zu fördern oder zu leiten. Weiterhin weist die Adaptionsleiste ein Adaptersperrelement auf, welches beweglich ausgeformt ist, um das Aufnahmeelement zum Aufstecken des Steckadapter des medizinischen Instruments freizugeben, wenn das Adaptersperrelement in einer ersten Stellung ist und/oder das Aufnahmeelement zu sperren, wenn das Adaptersperrelement in einer zweiten Stellung ist, wobei das Adaptersperrelement zumindest ein Sperrstück zum Sperren eines Durchflusses des Reinigungsmediums von dem Versorgungskanal die Medienleitung aufweist, das derart ausgebildet ist um in der ersten Stellung außerhalb der Medienleitung angeordnet zu sein, und in der zweiten Stellung innerhalb der Medienleitung angeordnet ist.

Gemäß einer Ausführungsform kann die Adaptionsleiste in einem Medienverteiler für das Reinigungsgerät angeordnet sein. Dabei kann die Adaptionsleiste beispielsweise zumindest teilweise aus Edelstahl ausgeformt sein, um Rost und eine damit eingehende Verschmutzung des Reinigungsmediums zu verhindern. Das Reinigungsgerät kann dabei als ein Dentalpflegegerät ausgebildet sein, welches medizinische Instrumente von außen wie innen erst reinigt und dann pflegt. Das Reinigungsgerät kann dabei mehrere, beispielsweise vier oder mehr Aufnahmeelemente aufweisen. Das Reinigungsgerät kann vorzugsweise mindestens auch bis zu zwölf Aufnahmeelemente aufweisen, um zwölf medizinische Instrumente aufzunehmen. Ein medizinisches Instrument kann als Beispiel ein Dentalbohrer, eine Dentalturbine und/oder ein Hand- und Winkelstück sein. Zur Aufnahme des medizinischen Instruments kann das Aufnahmeelement den Steckadapter mittels eines Ansteckens des medizinischen Instruments aufnehmen. Die Medienleitung sowie der Versorgungskanal können dabei beispielsweise ebenfalls aus Edelstahl ausgeformt sein, unter anderem auch um Produktionskosten niedrig zu halten oder gegenüber dem Reinigungsmedium nicht-korrosiv zu sein. Der Versorgungskanal kann ein Ölkanal oder ein Flottenkanal sein. Das Adaptersperrelement kann beispielsweise aus einem Blech oder Metall ausgeformt sein, um ein Bewegen des Adaptersperrelements zu erleichtern. Die erste Stellung des Adaptersperrelements kann dabei eine Stellung sein, in welcher das Aufnahmeelement und der Versorgungskanal verschlossen sind. Der Versorgungskanal kann von dem Sperrstück verschlossen werden, welches beispielsweise die Form eines Stifts, Steckelementes oder _{"}Verschluss-Korkens" hat. Die zweite Stellung kann dabei eingenommen werden, wenn ein medizinisches Instrument an dem Aufnahmeelement aufgesteckt ist, sodass das Reinigungsmedium durch das Aufnahmeelement fließen kann, um das medizinische Instrument zu reinigen. Durch das Adaptersperrelement und das Sperrstück kann das Aufnahmeelement und der Versorgungskanal gleichzeitig mit einer Bewegung verschlossen oder geöffnet werden.

Die Adaptionsleiste kann gemäß einer Ausführungsform ein Federelement umfassen, das ausgebildet ist, um das Adaptersperrelement in der zweiten Stellung als Ruhestellung zu halten. Das Federelement kann dabei als eine Sprungfeder ausgeformt sein. Das Federelement kann außerdem bis zu einem Abschlagpunkt zusammengedrückt werden, sodass das Federelement anschließend wieder in die ursprüngliche Form zurückspringen kann. Dabei kann das Federelement in einer Ruhestellung das Adaptersperrelement sicher geschlossen halten.

Das Federelement kann gemäß einer Ausführungsform auf einer dem Aufnahmeelement gegenüberliegenden Seite der Adaptionsleiste angeordnet sein. Hierdurch kann ein erforderlicher Platz im Bereich des Aufnahmeelementes für das aufzusteckende medizinische Instrument freigehalten werden. Auch kann durch diese Anordnung die Gefahr eines Verklemmens des Federelementes reduziert oder möglichst verhindert werden.

Die Adaptionsleiste kann außerdem gemäß einer Ausführungsform einen Bedienbügel aufweisen, der ausgebildet ist, um das Adaptersperrelement zwischen der ersten und der zweiten Stellung manuell zu bewegen. Der Bedienbügel kann dabei als ein Griff ausgeformt sein, um die Handhabung des Bügels bei einem Bewegen für einen Nutzer einfacher zu machen.

Das Adaptersperrelement kann gemäß einer Ausführungsform einen Vorsprung aufweisen, der sich zumindest teilweise über das Aufnahmeelement hinaus erstreckt. Der Vorsprung kann ein zu dem Aufnahmeelement gerichtetes Ende umfassen. Dadurch kann das Aufnahmeelement gesperrt werden, und nur dann geöffnet werden, wenn ein medizinisches Instrument aufgesteckt wird.

Der Vorsprung kann gemäß einer Ausführungsform ein rechtwinkliges Ende aufweisen, welches dazu ausgeformt ist, um eine Aufnahme des Steckadapters des medizinischen Instruments zu sperren. Das rechtwinklige Ende kann dabei eine gesamte Oberfläche des Aufnahmeelements abdecken, um zu verhindern, dass medizinische Instrumente ungewollt oder aus Versehen angebracht werden.

Alternativ kann der Vorsprung gemäß einer Ausführungsform ein zumindest teilweise schräges Ende, insbesondere an einer dem Aufnahmeelement zugewandten Seite, aufweisen, um das Aufnahmeelement zu sperren, insbesondere wobei das zumindest teilweise schräge Ende dazu ausgebildet ist, um das Adaptersperrelement in die zweite Stellung zu überführen, wenn der Steckadapter des medizinischen Instruments auf das Aufnahmeelement gesteckt wird. Dadurch wird eine halbautomatische Kanalfreigabe bei einem Aufstecken des medizinischen Instruments geschaffen, wodurch ein manuelles Bewegen des Adaptersperrelements nicht mehr von Nöten ist und der Nutzerkomfort bei der Verwendung einer derart ausgebildeten Adapterleiste erhöht wird.

Die Adaptionsleiste kann gemäß einer Ausführungsform auch einen weiteren Versorgungskanal aufweisen, von dem eine weitere Medienleitung zu dem Aufnahmeelement führt, wobei die weitere Medienleitung in der Adaptionsleiste ausgebildet ist, wobei das Adaptersperrelement ausgebildet ist, um in der zweiten Stellung die weitere Medienleitung mit einem weiteren Sperrstück zu verschließen. Hierdurch können beispielsweise mehrere verschiedene, in unterschiedlichen Kanälen geführte Medien in ein medizinisches Instrument geleitet werden, wodurch eine komfortable und flexible Reinigung und Pflege dieser medizinischen Instrumente ermöglicht wird. Insbesondere es sich dem ersten Versorgungskanal um einen Flottenkanal zur Zuleitung von Spülflotte und beim weiteren Kanal um einen Ölkanal zur Zuleitung von einem Pflegeöl handeln.

Das Sperrstück und das weitere Sperrstück können gemäß einer Ausführungsform beispielsweise eine unterschiedliche Länge aufweisen. Dadurch können die Medienleitungen hintereinander in der Adaptionsleiste geführt werden, was eine Realisierung einer platzsparenden Ausführungsform ermöglicht.

Außerdem kann die Adaptionsleiste gemäß einer Ausführungsform zudem einen Bypass-Kanal umfassen, der einen geringeren Querschnitt als die Medienleitung aufweist, um ein Fluid aus dem Versorgungskanal unter Umgehung des Sperrstücks an das Aufnahmeelement auszuleiten. Der Bypass-Kanal, der auch vereinfachend als Bypass bezeichnet werden kann, kann dabei aus hygienischen Gründen vorhanden sein, um beispielsweise ein in dem Versorgungskanal länger vorgehaltenes Reinigungsmittel aus dem Versorgungskanal auszulassen und hierdurch Hygienevorschriften zu entsprechen.

Gemäß einer vorteilhaften Ausführungsform kann die Adaptionsleiste zumindest ein zusätzliches Aufnahmeelement für einen zusätzlichen Steckadapter aufweisen, welches dazu ausgeformt ist, um ein zusätzliches medizinisches Instrument aufzunehmen. Die Adaptionsleiste kann zumindest eine zusätzliche Medienleitung aufweisen, die in der Adaptionsleiste ausgebildet ist, welche mit dem Versorgungskanal verbunden ist, um das Reinigungsmedium zu dem zusätzlichen medizinischen Instrument zu fördern oder zu leiten. Weiterhin kann die Adaptionsleiste ein zusätzliches Adaptersperrelement aufweisen, welches beweglich ausgeformt ist, um das zusätzliche Aufnahmeelement zum Aufstecken des zusätzlichen Steckadapters des medizinischen Instruments freizugeben, wenn das zusätzliche Adaptersperrelement in einer ersten Stellung ist und/oder das zusätzliche Aufnahmeelement zu sperren, wenn das zusätzliche Adaptersperrelement in einer zweiten Stellung ist. Dabei umfasst das zusätzliche Adaptersperrelement zumindest ein zusätzliches Sperrstück, das derart ausgebildet ist. um in der ersten Stellung außerhalb der zusätzlichen Medienleitung angeordnet zu sein und in der zweiten Stellung innerhalb der zusätzlichen Medienleitung angeordnet zu sein. Durch eine derartige Ausführungsform kann die Adaptionsleiste zur Reinigung von mehreren medizinischen Instrumenten verwendet werden, sodass die Aufnahmeelemente einfach und komfortabel verschlossen oder bei einem entsprechenden aufgesteckten Instrument geöffnet werden können.

Es wird ferner ein Reinigungsgerät zur Reinigung von zumindest einem medizinischen Instrument mit einer Adaptionsleiste vorgestellt. Das Reinigungsgerät kann dabei ein Pflegegerät, insbesondere ein Dentalpflegegerät sein. Eine solche Ausführungsform ermöglicht ebenfalls eine schnelle, ressourcenschonende und hygienische Reinigung von medizinischen Instrumenten.

Der beschriebene Ansatz wird anhand eines gewerblichen oder professionellen Geräts, beispielsweise einem medizinischen Gerät, wie einem Reinigungs- oder Desinfektionsgerät, einem Kleinsterilisator, einem Großraumdesinfektor oder einer Container-Waschanlage beschrieben, wobei jedoch auch andere Einsatzgebiete prinzipiell denkbar sin.

Ein Ausführungsbeispiel der Erfindung ist in den Zeichnungen rein schematisch dargestellt und wird nachfolgend näher beschrieben. Es zeigt
- Figur 1: eine schematische Darstellung eines Ausführungsbeispiels eines Reinigungsgeräts;
- Figur 2: eine schematische Perspektivdarstellung eines Ausführungsbeispiels einer Adaptionsleiste;
- Figur 3: eine weitere schematische Perspektivdarstellung eines Ausführungsbeispiels einer Adaptionsleiste mit einem aufgesteckten medizinischen Instrument;
- Figur 4: eine Schnittdarstellung eines Ausführungsbeispiels einer Adaptionsleiste;
- Figur 5: eine weitere Schnittdarstellung eines Ausführungsbeispiels einer Adaptionsleiste mit einem aufgesteckten medizinischen Instrument;
- Figur 6: eine weitere Schnittdarstellung eines weiteren Ausführungsbeispiels einer Adaptionsleiste; und
- Figur 7: eine weitere Schnittdarstellung eines Ausführungsbeispiels einer Adaptionsleiste mit einem aufgesteckten medizinischen Instrument.

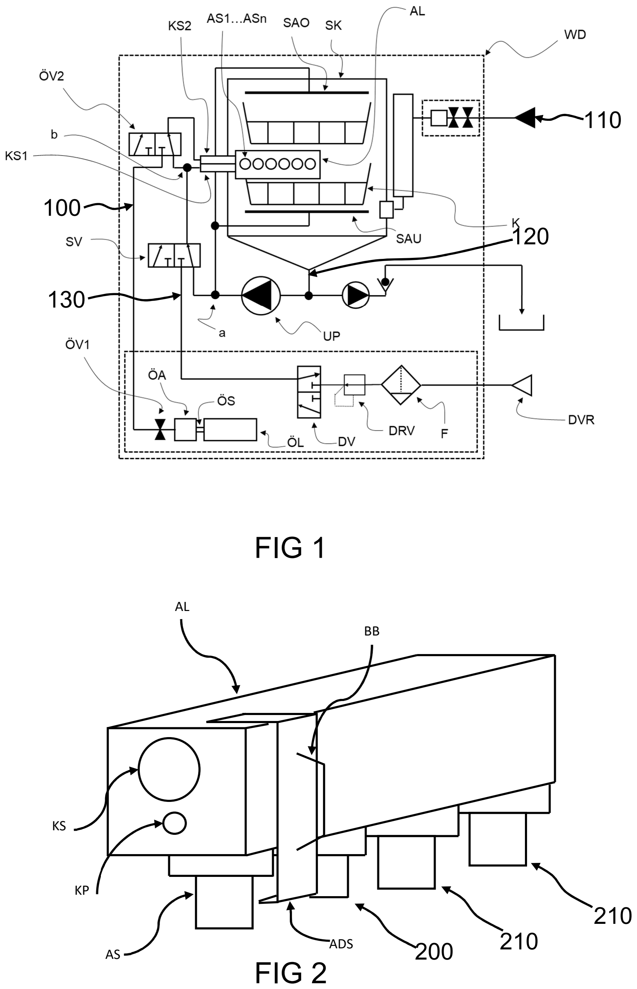

Figur 1 zeigt eine schematische Darstellung eines Ausführungsbeispiels eines Reinigungsgeräts WD. Das Reinigungsgerät WD umfasst einen Reinigungsraum SK mit einen Sprüharm SAO, einen weiteren Sprüharm SAU, ein Verteilelement VK mit verschiedenen Anschlusspositionen AS1, ASn und einen Korb K. Eine Pflegemittelleitung 100 mit einer Pflegemittelbereitstellungseinheit ÖL ermöglicht eine Leitung von Pflegemittel über ein Pflegemittelventil ÖV2 an eine zweite Korbschnittstelle KS2 des Reinigungsraums SK. Ein Reinigungsmittelanschluss 110 ermöglicht die Leitung des Reinigungsmittels in das Reinigungsgerät WD, wobei das Reinigungsmittel durch eine Reinigungsmittelfördereinrichtung UP verteilbar ist. Das Reinigungsmittel wird dabei durch eine Reinigungsmittelleitung 120 in den Reinigungsraum SK geleitet. Dabei ist die Reinigungsmittelleitung 120 und eine Druckluftleitung 130 über das Spülmittelventil SV gekoppelt. Zusätzlich umfasst das Reinigungsgerät WD eine Druckluftbereitstellungseinheit DVR, die Druckluft zu dem Verteilelement VK zu leitet.

Gemäß einem Ausführungsbeispiel wird das Reinigungsmittel nun durch das Pflegemittelmittelventil ÖV2 an die zweite Korbschnittstelle KS2 geleitet. In dem Reinigungsraum SK wird das Reinigungsmittel dann in das Verteilelement VK geleitet. Dabei hat das Reinigungsmittel nicht nur die Funktion die medizinischen Instrumente in dem Reinigungsraum SK zu reinigen, sondern auch die Reinigungsmittelleitung 120 zu säubern.

Das Pflegemittel wird dabei nach einem Reinigungsvorgang von der Pflegemittelbereitstellungseinheit ÖL in die Pflegemittelleitung 100 gegeben. Die Pflegemittelbereitstellungseinheit ÖL ist dabei beispielsweise als eine Ölkartusche oder eine Öldose mit Treibgas ausgeformt. Dabei wird das Pflegemittel erst von einem Ölstößel ÖS in eine Ölkartuschenadaption ÖA gegeben, welche schließlich das Pflegemittel ausgibt. Um zu verhindern, dass das Pflegemittel zurück in die Ölkartuschenadaption ÖA fließt, ist ein Ölventil ÖV1 in der Pflegemittelleitung 100 angeordnet.

Gemäß einem Ausführungsbeispiel ist die Druckluftbereitstellungseinheit DVR dazu vorgesehen, um Druckluft in die Druckluftleitung 130 des Reinigungsgeräts WD zu leiten. Die Druckluftbereitstellungseinheit DVR ist dabei als ein Druckluftversorgungsanschluss des Reinigungsgeräts WD ausgebildet. Dabei wird die Druckluft in dem Reinigungsgerät WD zuerst von einem Filter F gereinigt, um zu verhindern, dass Keime über diesen Weg in das Reinigungsgerät WD gelangen. Um einen stetigen Druck bereitzustellen, weist das Reinigungsgerät WD ein Druckregelventil DRV auf. Dabei wird ein Druck von beispielsweise 3 bis 5 bar durch das Druckregelventil DRV passieren gelassen. Anschließend wird die Druckluft durch ein Druckluftventil DV geleitet. Entweder wird die Druckluft dann zu dem Spülmittelventil SV geleitet oder aber aus dem Reinigungsgerät WD abgeleitet, um einen Druckstau zu vermeiden und eine Entlüftung vorzunehmen.

Sowohl das Pflegemittel, das Reinigungsmittel oder die Druckluft, werden durch die erste Korbschnittstelle KS1 oder die zweite Korbschnittstelle KS2 in den Reinigungsraum SK geleitet. Die erste Korbschnittstelle KS1 und die zweite Korbschnittstelle KS2 sind dabei Koppelstellen für das Verteilelement VK. Das Verteilelement VK ist dabei eine Verteilkammer mit einer Adaptionsleiste AL welche mehrere Anschlusspositionen AS1, ASn aufweist. An den Anschlusspositionen AS1, ASn können hier jeweils ein medizinisches Instrument angeschlossen werden, um diese von innen zu reinigen und zu pflegen. Um medizinische Instrumente von außen zu reinigen und zu pflegen, hat der Reinigungsraum SK dem Korb K. Gemäß einem Ausführungsbeispiel hat das Reinigungsgerät WD auch einen weiteren Korb.

Die Adaptionsleiste AL ist zur Aufnahme mindestens eines medizinischen Instruments ausgeformt, wobei die Adaptionsleiste AL zumindest ein Aufnahmeelement für einen Steckadapter umfasst, welches dazu ausgeformt ist, um das medizinische Instrument durch ein Aufstecken aufzunehmen. Die Adaptionsleiste AL weist zumindest eine Medienleitung auf, die in der Adaptionsleiste AL ausgebildet ist, welche mit einem Versorgungskanal verbunden ist, um Reinigungsmedien zu dem medizinischen Instrument zu fördern und ein Adaptersperrelement, welches beweglich ausgeformt ist, um das Aufnahmeelement zum Aufstecken des Steckadapter des medizinischen Instruments freizugeben, wenn sich das Adaptersperrelement in einer ersten Stellung befindet und/oder das Aufnahmeelement zu sperren, wenn sich das Adaptersperrelement in einer zweiten Stellung befindet, wobei das Adaptersperrelement zumindest ein Sperrstück zum Sperren eines Durchflusses des Reinigungsmediums von dem Versorgungskanal zu einer Medienleitung aufweist, wobei das Sperrstück derart ausgebildet ist, um in der ersten Stellung außerhalb der Medienleitung angeordnet zu sein, und in der zweiten Stellung innerhalb der Medienleitung angeordnet ist.

Die Adaptionsleiste umfasst eine halbautomatische oder auch eine manuelle Prozessmedienfreigabe im Korbeinsatz zur Aufbereitung und automatisierten Pflege von Dentalinstrumenten im Reinigungs- und Desinfektionsgerät.

Bei der automatisierten Aufbereitung mit integrierter Instrumentenpflege von Hand- und Winkelstücken bzw. Dentalturbinen in einem Reinigungs- und Desinfektionsgerät können diese gemäß der Erfindung mit Spülflotte und Pflegeöl versorgt werden. Dies geschieht beispielsweise über einen Korbeinsatz bzw. die Adaptionsleiste, an welchem/welcher die Instrumente mit Steckadaptern verbunden werden, um so die Innenkanäle dieser medizinischen Instrumente zu reinigen, aufzubereiten und zu pflegen. Der Korbeinsatz bzw. die Adaptionsleiste ist seinerseits/ihrerseits mit innenliegenden Versorgungskanälen versehen, welche die Prozess- bzw. Reinigungsmedien zu den entsprechenden Innenkanälen der Steckadapter leiten. Dabei sind, in einer Ausprägung des Einsatzes beispielsweise insgesamt acht Adaptionsplätze zu je zwei Adaptionsleisten mit jeweils vier Adaptionsplätzen vorgesehen und hintereinander angeordnet.

Gegenüber üblichen Korbeinsätzen entfällt mit dem hier vorgestellten Ansatz somit der Nachteil, dass auch bei einer Teilbestückung des Korbeinsatzes bzw. der Adaptionsleiste, Spülflotte und Pflegeöl an den nicht bestückten Adaptern austritt. Dies hätte einen verminderten Spüldruck innerhalb der Instrumente während der Aufbereitung bzw. Reinigung zur Folge, welcher durch eine erhöhte Förderleistung respektive Drehzahl der Umwälzpumpe ausgeglichen werden müsste. Dies ist aus prozesstechnischen Gründen nicht immer möglich. Zudem tritt in derartigen Lösungen, wenngleich auch nur eine sehr geringe Menge pro Adaptionsplatz, Pflegeöl aus den unbestückten Aufnahmeelementen aus.

Diesem Umstand soll der hier beschriebene Ansatz durch einen manuellen bzw. halbautomatischen und beim Aufstecken des medizinischen Instrumentes auf den Aufnahmeelement betätigten Mechanismus begegnen, welcher den Weg der Prozessmedien im jeweiligen Aufnahmeelement nur dann freigibt, wenn dieses auch bestückt ist. So wird der Spüldruck in den Instrumenten bei geringer Bestückungszahl und gleichbleibender Pumpendrehzahl erhöht, was die Reinigungs- und Desinfektionsleistung verbessert. Zugleich wird das Pflegeöl effizienter genutzt und es kommt zu einer erheblichen Verringerung von Ölanhaftungen auf eventuell gleichzeitig im Spülraum befindlichem Spülgut. Da die ins Instrument eingebrachte Ölmenge nicht erfasst wird und die Absicherung des Eintrags einer Mindestmenge des Pflegeöls nur über die Prozessparameter abgesichert wird, verringert der hier vorgestellte Ansatz das Risiko einer zu gering dosierten Pflegeölmenge in einem Instrument.

Die Prozessmedien folgen nun nicht mehr dem Weg des geringsten hydraulischen bzw. pneumatischen Widerstands- also dem Weg zu einem unbestückten und somit offenen Aufnahmeelement- sondern werden effizient und zielgerichtet an die jeweiligen Aufnahmeelemente geführt.

Der hier vorgestellte Ansatz soll bewusst eine manuelle Betätigung zur Freigabe der Prozessmedien für einen bestimmten Adaptionsplatz ermöglichen, da eine elektronische, pneumatische oder hydraulische Betätigung innerhalb oder außerhalb des Spülraumes mit entsprechenden Zuleitungen und Positionsabfragen für die eingenommene Schaltstellung konstruktiv sehr komplex und somit unwirtschaftlich ist. Es werden folglich keine im Spülraum befindlichen Aktoren zur Freigabe oder Abschaltung von Prozessmedien in oder an den jeweiligen Instrumenteneinsätzen verwendet.

Ein ähnliches System zur Trennung von Kanälen im Zuge eines Aufbereitungsvorganges kann im Kontext der Aufbereitung von flexiblen Endoskopen als medizinischen Instrumenten verwendet werden. Dort können die Innenkanäle von Endoskopen zwischen Anschlussstecker und distalem Ende zur Probenentnahme während der Endoskopie am Bedienteil geöffnet bzw. zur Aufbereitung wieder verschlossen werden.

Zudem wird die Menge des Pflegeöls, welches tatsächlich in das Instrument eingetragen wird in keinem Gerät überwacht bzw. erfasst. Aus diesem Grund sichern die meisten Hersteller der Aufbereitungsgeräte mit integrierter automatischer Instrumentenpflegefunktion dieses Risiko durch eine Überölung, also einer überhöhten Beaufschlagung des Instrumentes mit dem Pflegemedium ab. Dies führt zu einem unnötig hohen Ölverbrauch bzw. Öleintrag ins Abwasser und einer äußeren Benetzung des übrigen Spülgutes bzw. der umliegenden Instrumente.

Der hier vorgestellte Ansatz steigert somit die Reinigungs- und Hygieneleistung des Reinigungs- und Desinfektionsgerät mit Dentalinstrumenteneinsatz erheblich, da sie die durch nicht angeschlossene Instrumente offenen und so im hydraulischen System als Leckage wirkenden Anschlüsse fast vollständig schließt und so den Spüldruck in den angeschlossenen Instrumenten steigert. Zudem reduziert der hier vorgestellte Ansatz den Ölverbrauch, die Benetzung anderer im Spülraum befindlicher Instrumente und den Eintrag von Pflegeöl ins Abwasser.

Figur 2 zeigt eine schematische Perspektivdarstellung eines Ausführungsbeispiels einer Adaptionsleiste AL. Die Adaptionsleiste AL ist dabei als eine Adaptionsleiste ausgeformt, wie sie gemäß einem Ausführungsbeispiel des Reinigungsgerätes aus Figur 1 verwendet werden kann. Die Adaptionsleiste AL hat hierbei einen Versorgungskanal KS und einen weiteren Versorgungskanal KP. Außerdem umfasst die Adaptionsleiste AL zumindest ein Aufnahmeelement AS, zur Aufnahme von medizinischen Instrumenten und ein Adaptersperrelement ADS mit einem Bedienbügel BB. Das Adaptersperrelement ADS ist dazu vorgesehen, um das Aufnahmeelement AS zu sperren.

Gemäß dem hier gezeigten Ausführungsbeispiel umfasst die Adaptionsleiste AL insgesamt vier Aufnahmen, das als Aufnahmeelement AS bezeichnet wird, ein zusätzliches Aufnahmeelement 200, und zwei weitere Aufnahmeelemente 210. Das Aufnahmeelement AS und das zusätzliche Aufnahmeelement 200, und die zwei weiteren Aufnahmeelemente 210 sind dabei gleich ausgeformt. Dabei ist das Aufnahmeelement AS zur Aufnahme eines Steckadapters eines medizinischen Elements ausgeformt. Das heißt auf das Aufnahmeelement AS wird ein medizinisches Instrument aufgeschoben oder aufgesteckt. Der Versorgungskanal KS und der weitere Versorgungskanal KP leiten dabei Fluide zur Reinigung und/oder Instandhaltung/Pflege in die Adaptionsleiste AL, wobei in der Adaptionsleiste AL eine Medienleitung für ein Reinigungsmedium und eine weitere Medienleitung bei für ein Pflegemedium oder ein weiteres Reinigungsmedium ausgeformt sind, welche die Fluide weiterleiten oder führen. Genauer versorgt der Versorgungskanal KS die Adapterleiste AL mit Reinigungsmittel, einer Spülflotte oder mit Druckluft. Der weitere Versorgungskanal KP versorgt die Adaptionsleiste AL wiederum mit Pflegemittel, welches beispielsweise ein Pflegeöl, wie etwa Weißöl, sein kann.

Die Adaptionsleiste AL mit vier Aufnahmen hat hier eine manuelle Kanalfreigabe, beispielhaft an einer Adapterposition in welcher ein Adapter gesperrt ist. Der Adapter ist dabei als Adaptersperrelement ADS ausgeformt. Die Kanalfreigabe ist manuell bedienbar, da das Adaptersperrelement ADS den Bedienbügel BB umfasst, welcher einem Nutzer ermöglicht das Adaptersperrelement ADS manuell zu bewegen, um ein Medienabgabe über das Aufnahmeelement AS freizugeben. Ist das Adaptersperrelement ADS in einer geschlossenen Stellung, oder auch einer zweiten Stellung, welche als Ruheposition für das Adaptersperrelement ADS fungiert, ist die Medienabgabe über das Aufnahmeelement AS gesperrt.

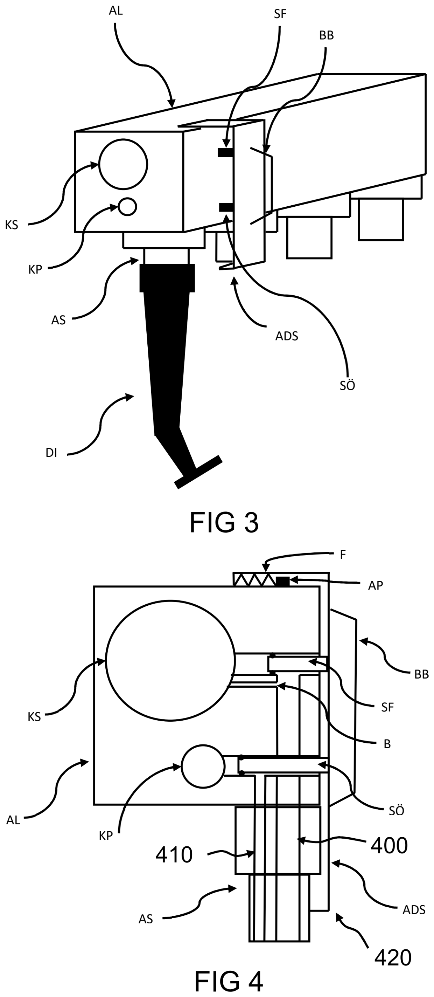

Figur 3 zeigt eine weitere schematische Perspektivdarstellung eines Ausführungsbeispiels einer Adaptionsleiste AL mit einem medizinischen Instrument DI. Die Adaptionsleiste AL ist gemäß einem Ausführungsbeispiel wie die in Figur 1 beschriebene Adaptionsleiste ausgeformt. Das Adaptersperrelement ADS befindet sich dabei in der ersten Stellung, in welcher das Aufnahmeelement AS freigegeben ist, beziehungsweise hier das medizinische Instrument DI daran angebracht ist. Ebenfalls zu sehen ist ein Sperrstück SF und ein weiteres Sperrstück SÖ, welche an dem Adaptersperrelement ADS angebracht sind.

Gemäß einem Ausführungsbeispiel ist das medizinische Instrument DI, welches hier an dem Aufnahmeelement AS aufgesteckt ist, als ein Dentalinstrument inklusive einem Steckadapter ausgeformt. Das medizinische Instrument DI kann dabei nur angebracht werden, da das Adaptersperrelement ADS in der ersten Stellung verbracht ist. In der ersten Stellung sind dabei das Sperrstück SF und das weitere Sperrstück SÖ nicht mehr vollständig in der Adaptionsleiste AL eingeführt, und sind sichtbar. Das Sperrstück SF ist dabei dazu vorgesehen den Versorgungskanal KS in der ersten Stellung des Adaptersperrelements ADS freizugeben und das weitere Sperrstück SÖ gibt in der ersten Stellung in der ersten Stellung des Adaptersperrelements ADS den weiteren Versorgungskanal KP frei.

Das Adaptersperrelement ADS lässt sich dabei manuell mit dem Bedienbügel BB von der ersten in die zweite Stellung und von der zweiten in die erste Stellung überführen, wodurch der Nutzer genau festlegt, ob das Aufnahmeelement AS gesperrt oder freigegeben ist. Wenn die Adapterleiste AL mehrere Aufnahmen, wie hier beispielsweise vier, umfasst, hat jedes weitere Aufnahmeelement beispielsweise einen jeweiligen eigenen Bedienbügel.

Der hier vorgestellte Ansatz bildet beispielsweise eine Ausprägung einer Adaptionsleiste AL, in welchem alle Aufnahmeelemente AS gleichermaßen und über den gesamten Aufbereitungs- und Pflegeprozess hinweg Prozessmedien zugeführt werden. Durch die Einbringung von dem Sperrstück SF und dem weiteren Sperrstück SÖ in den Versorgungskanal KS und den weiteren Versorgungskanal KP kann eine entsprechende Abgabe von Medien unterbrochen werden. Das Sperrstück SF und das weitere Sperrstück SÖ sind an einem Adaptersperrelement ADS angebracht, welches in der Ausprägung durch seine, dem Instrumentenadapter vorgelagerte Geometrie verhindert, dass dieser aufgesteckt und somit hydraulisch mit dem Aufbereitungssystem verbunden wird. Zudem sind das Sperrstück SF und das weitere Sperrstück SÖ mit Dichtelementen (hier O-Ringe) und einem Bedienbügel BB zur linearen Verschiebung versehen.

Figur 4 zeigt eine Schnittdarstellung eines Ausführungsbeispiels einer Adaptionsleiste AL. Die Adaptionsleiste AL ist dabei gemäß einem Ausführungsbeispiel wie die in Figur 2 oder 3 beschriebene Adaptionsleiste ausgeformt. Es werden dabei in der Figur 4 eine Medienleitung 400, eine weiterer Medienleitung 410 und deren Verbindung zu dem Versorgungskanal KS und dem weiteren Versorgungskanal KP gezeigt. Ebenfalls zu sehen sind ein Federelement F und ein Anschlagpunkt AP auf einer hier oben dargestellten Seite der Adaptionsleiste AL.

Die Medienleitung 400 ist derart angeordnet, dass sie eine Verbindung zwischen dem Aufnahmeelement AS und dem Versorgungskanal KS bildet, wobei über den Versorgungskanal KS Spülmittelflotte für die Adaptionsleiste AL bereitgestellt werden kann. Hier wird der Versorgungskanal KS allerdings von dem Sperrstück SF versperrt, da sich das Adaptersperrelement ADS in der zweiten Stellung befindet. Nicht abgesperrt von dem Sperrstück SF wird ein Bypass-Kanal B, welcher es ermöglicht, dass eine kleine Menge, beispielsweise 10 Milliliter, zu Reinigungs- oder Hygienezwecken aus dem Versorgungskanal KS in Richtung zu dem Aufnahmeelement austreten kann. Ebenfalls versperrt wird der weitere Versorgungskanal KP von dem weiteren Sperrstück SF, damit kein Pflegemittel in die weitere Medienleitung 410 passieren kann. Hierbei verläuft die Medienleitung 400 versetzt zu der weiteren Medienleitung 410, sodass sich diese nicht kreuzen und die darin geführten Medien voneinander getrennt bleiben.

Gemäß dem hier gezeigte Ausführungsbeispiel umfasst das Adaptersperrelement ADS einen Vorsprung 420 an einem dem Aufnahmeelement AS zugewandten Ende des Adaptersperrelements ADS. Dieser Vorsprung 420 weist beispielsweise eine rechtwinklige Kante auf, welche in einem geschlossenen Zustand ein Aufstecken eines medizinischen Instrumentes auf das Aufnahmeelement AS versperrt.

Um das Adaptersperrelement ADS in die erste Position zu bewegen, bzw. zu öffnen, kann beispielsweise durch einen Benutzer an dem Bedienbügel BB gezogen werden. Dabei wird das Federelement F zwischen einem dem Vorsprung 420 gegenüberliegenden Ende und dem Anschlagpunkt AP zusammengedrückt. Die Länge des Federelements F beschränkt dabei, wie weit das Adaptersperrelement ADS bewegt werden kann. Wenn das Adaptersperrelement ADS wieder in die Ruheposition gedrückt wird, entspannt sich das Federelement F wieder.

In diesem als "gesperrt" beschriebenen Zustand kann nur ein sehr geringer Teil der Spülflotte über den Bypass-Kanal B an dem Sperrstück SF vorbeifließen. Dies ist aus hygienischen Gründen sinnvoll. Allerdings ist dieser Anteil gemessen an Gesamtvolumenstrom es Spülflottenkreislaufes zu vernachlässigen. Ein so gesperrter Anschluss verringert die gegenüber dem Stand der Technik entstehenden Leckagen auf ein absolutes Minimum und erhöht so, bei gleichbleibendem Volumenstrom im Umwälzkreislauf, den Spüldruck in das "freigegebenen" Aufnahmeelement AS. Ebenso wird die über den weiteren Versorgungskanal KP eingespeiste Menge an Pflegemittel bzw. Öl deutlich effektiver in die Instrumente eingebracht, da der nicht mehr an den offenen und unbesetzten Adaptionsplätzen austritt, sondern in die angeschlossenen und freigegebenen Instrumente geleitet wird. In beiden Fällen wird das Adaptersperrelement ADS von dem Federelement F zurück in seine Ausgangsposition gedrückt. So ist ein unbeabsichtigt geöffneter Adaptionsanschluss ausgeschlossen, welcher zu den genannten Nachteilen führt Der hier vorgestellte Ansatz wird hier in zwei Ausführungsformen beschrieben, wobei der zuvor erläuterte Teil die über beide Ausprägungen hinweg identischen inneren mechanischen und hydraulischen Zustände und konstruktiven Umsetzungen beinhaltet.

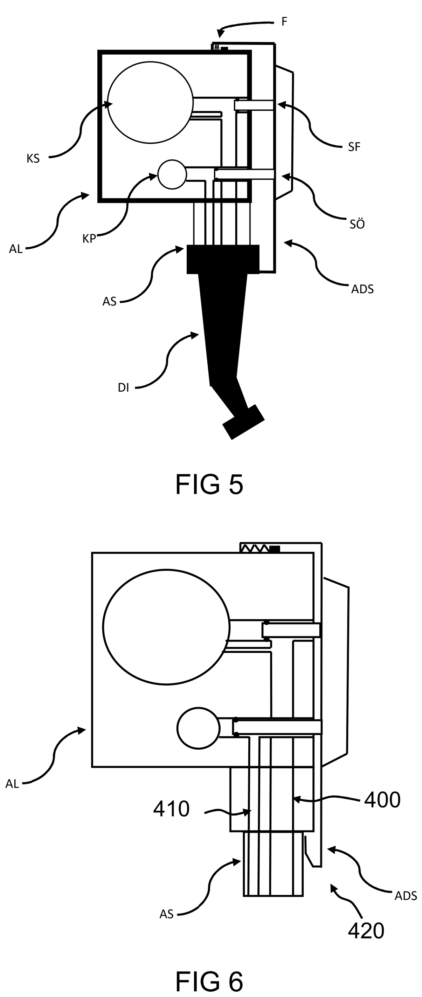

Figur 5 zeigt eine Schnittdarstellung eines Ausführungsbeispiels einer Adaptionsleiste AL mit einem medizinischen Instrument DI. Die Adaptionsleiste AL ist dabei gemäß einem Ausführungsbeispiel wie die in Figur 2 oder 3 beschriebene Adaptionsleiste ausgeformt. Das Adaptersperrelement ADS ist in der ersten Stellung und das medizinische Element DI ist auf dem Aufnahmeelement AS aufgesteckt.

Durch die erste Stellung, welche das Adaptersperrelement ADS einnimmt, wird das Federelement F zusammengedrückt, das Sperrstück SF und das weitere Sperrstück SÖ geben hier den Versorgungskanal KS und den weiteren Versorgungskanal KP frei. Zusätzlich ist das Aufnahmeelement AS nicht weiter versperrt und das medizinische Instrument DI ist aufgesteckt.

Durch das Zurückziehen des Adaptersperrelements ADS am Bedienbügel BB bis zu einem definierten und mechanisch festgelegten Anschlagpunkt AP werden das Sperrstück SF und das weitere Sperrstück SÖ aus dem Versorgungskanal KS und dem weiteren Versorgungskanal KP ebenfalls zurückbewegt und geben so den Medienweg für den entsprechenden Adaptionsplatz frei. Zeitgleich wird der Steckadapter des medizinischen Instrumentes DI freigegeben und das medizinische Instrument DI lässt sich auf die Steckadapteraufnahme AS aufstecken. Ein solcher Adaptionsplatz gilt als "freigegeben".

Figur 6 zeigt eine Schnittdarstellung eines weiteren Ausführungsbeispiels einer Adaptionsleiste AL. Die Adaptionsleiste AL ist dabei gemäß einem Ausführungsbeispiel wie die in Figur 4 beschriebene Adaptionsleiste ausgeformt. Das Adaptersperrelement ADS ist hier wieder in der zweiten Stellung wiedergegeben und es ist kein Medienfluss in der Adaptionsleiste AL vorhanden.

Gemäß diesem Ausführungsbeispiel weist der Vorsprung 420 kein gerades Ende auf, sondern an einer inneren Seite ein leicht schräges Ende. Dadurch wird das Adaptersperrelement ADS automatisch, oder halbautomatisch, zur Seite in die erste Stellung geschoben, wenn ein medizinisches Element manuell auf das Aufnahmeelement AS aufgesteckt wird. Auch die Medienleitung 400 und die weitere Medienleitung 410 werden somit geöffnet.

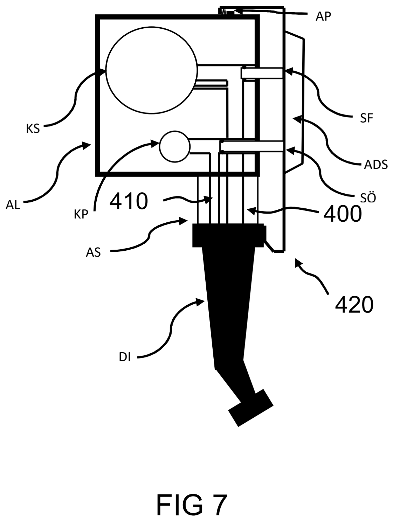

Figur 7 zeigt eine Schnittdarstellung des weiteren Ausführungsbeispiels der Adaptionsleiste AL mit einem aufgesteckten medizinischen Instrument DI. Die Adaptionsleiste AL ist dabei gemäß einem Ausführungsbeispiel wie die in Figur 6 beschriebene Adaptionsleiste ausgeformt. Im Unterschied zu der in Figur 4 beschriebenen Adaptionsleiste AL weist nun der Vorsprung 420 ein zumindest teilweise schräges Ende auf, wie zur Figur 6 bereits beschrieben wurde.

Durch das aufgesteckte medizinische Instrument DI ist das Adaptersperrelement ADS in der ersten Stellung und die Medienleitung 400 und die weitere Medienleitung 410 sind wieder freigegeben und leiten Reinigungsmittel und Pflegemittel (beispielsweise nacheinander) in das medizinische Instrument DI.

In dieser Ausprägung wird das Adaptersperrelement ADS durch den Aufsteckvorgang des Instrumentenadapters DI selbst bis zu einem definierten und mechanisch festgelegten Anschlagpunkt AP zurückgeschoben. Die Bewegung des Adaptersperrelements ADS, hier ein Sperrblech und damit auch die Bewegung. Das Sperrstück SF und das weitere Sperrstück SÖ in den Versorgungskanal KS und den weiteren Versorgungskanal KP wird durch eine Schräge am unteren Ende desselben eingeleitet. Dort schiebt der Instrumentenadapter das Blech zur Seite und gibt so den Medienweg für den entsprechenden Adaptionsplatz frei. Ein solcher Adaptionsplatz gilt ebenfalls als "freigegeben".

Es ergibt sich bei einem nicht vollständig bestückten Korbeinsatz zur Aufbereitung von medizinischen Instrumenten eine erheblich gesteigerte Reinigungs- und Desinfektionsleistung in den Innenkanälen der medizinischen Instrumente, d.h. der Hand- und Winkelstücke bzw. der Dentalturbinen bei gleichzeitiger Nutzung der Umwälzpumpe in ihrem effizientesten Arbeitspunkt. Zudem wird die Nutzungseffizienz des Pflegemediums gesteigert und die davon ausgehenden Restmengen bzw. Anhaftungen auf anderen Instrumenten innerhalb des Spülraumes stark reduziert.

Diese Eigenschaften führen zu einem besseren Aufbereitungsergebnis, einer verlängerten Einsatzzeit eines Pflegeöldruckgebindes und zu einer verringerten Umweltbelastung.

## Patentansprüche

1. Adaptionsleiste (AL) für ein Reinigungsgerät (WD) zur Aufnahme mindestens eines medizinischen Instruments (DI), insbesondere für ein Dentalpflegegerät, wobei die Adaptionsleiste (AL) folgende Merkmale aufweist:
zumindest ein Aufnahmeelement (AS) für einen Steckadapter, welches dazu ausgeformt ist, um das medizinische Instrument (DI) aufzunehmen;
zumindest eine Medienleitung (400) die in der Adaptionsleiste (AL) ausgebildet ist, welche mit einem Versorgungskanal (KS) verbunden ist, um ein Reinigungsmedium zu dem medizinischen Instrument (DI) zu fördern oder zu leiten; und
ein Adaptersperrelement (ADS), welches beweglich ausgeformt ist, um das Aufnahmeelement (AS) zum Aufstecken des Steckadapter des medizinischen Instruments (DI) freizugeben, wenn das Adaptersperrelement (ADS) in einer ersten Stellung ist und/oder das Aufnahmeelement (AS) zu sperren, wenn das Adaptersperrelement (ADS) in einer zweiten Stellung ist, wobei das Adaptersperrelement (ADS) zumindest ein Sperrstück (SF) zum Sperren eines Durchflusses des Reinigungsmediums von dem Versorgungskanal (KS) die Medienleitung (400) aufweist, das derart ausgebildet ist, um in der ersten Stellung außerhalb der Medienleitung (400) angeordnet zu sein, und in der zweiten Stellung innerhalb der Medienleitung (400) angeordnet ist.

2. Adaptionsleiste (AL) gemäß Anspruch 1, mit einem Federelement (F), das ausgebildet ist, um das Adaptersperrelement (ADS) in der zweiten Stellung als Ruhestellung zu halten.

3. Adaptionsleiste (AL) gemäß Anspruch 2, wobei das Federelement (F) auf einer dem Aufnahmeelement (AS) gegenüberliegenden Seite der Adaptionsleiste (AL) angeordnet ist.

4. Adaptionsleiste (AL) gemäß einem der vorangegangenen Ansprüche, mit einem Bedienbügel (BB), der ausgebildet ist, um eine manuelle Bewegung des Adaptersperrelements (ADS) zwischen der ersten und der zweiten Stellung zu ermöglichen.

5. Adaptionsleiste (AL) gemäß einem der vorangegangenen Ansprüche, wobei das Adaptersperrelement (ADS) einen Vorsprung (420) aufweist, der sich zumindest teilweise über das Aufnahmeelement (AS) hinaus erstreckt.

6. Adaptionsleiste (AL) gemäß Anspruch 5, wobei der Vorsprung (420) ein rechtwinkliges Ende aufweist, welches dazu ausgeformt ist, um eine Aufnahme des Steckadapters des medizinischen Instruments (DI) zu sperren.

7. Adaptionsleiste (AL) gemäß einem der vorangegangenen Ansprüche, wobei der Vorsprung (420) ein zumindest teilweise schräges Ende, insbesondere an einer dem Aufnahmeelement (AS) zugewandten Seite, aufweist, um das Aufnahmeelement (AS) zu sperren, insbesondere wobei das zumindest teilweise schräge Ende ausgebildet ist, um das Adaptersperrelement (ADS) in die erste Stellung zu überführen, wenn der Steckadapter des medizinischen Instruments (DI) auf das Aufnahmeelement (AS) gesteckt wird.

8. Adaptionsleiste (AL) gemäß einem der vorangegangenen Ansprüche, mit einem weiteren Versorgungskanal (KP) von dem eine weitere Medienleitung (410) zu dem Aufnahmeelement (AS) führt, wobei die weitere Medienleitung (410) in der Adaptionsleiste (AL) ausgebildet ist, wobei das Adaptersperrelement (ADS) ausgebildet ist, um in der zweiten Stellung die weitere Medienleitung (410) mit einem weiteren Sperrstück (SÖ) zu verschließen.

9. Adaptionsleiste (AL) gemäß Anspruch 8, wobei das Sperrstück (SF) und das weitere Sperrstück (SÖ) eine unterschiedliche Länge aufweisen.

10. Adaptionsleiste (AL) gemäß einem der vorangegangenen Ansprüche, mit einem Bypass-Kanal (B) der einen geringeren Querschnitt als die Medienleitung (400) aufweist, um ein Fluid aus dem Versorgungskanal (KS) unter Umgehung des Sperrstücks (SF) an das Aufnahmeelement (AS) auszuleiten.

11. Adaptionsleiste (AL) gemäß einem der vorangegangenen Ansprüche, mit zumindest einem zusätzlichen Aufnahmeelement (200) für einen zusätzlichen Steckadapter, welches dazu ausgeformt ist, um ein zusätzliches medizinisches Instrument aufzunehmen,
wobei die Adaptionsleiste (AL) zumindest eine zusätzliche Medienleitung aufweist, die in der Adaptionsleiste (AL) ausgebildet ist, welche mit dem Versorgungskanal (KS) verbunden ist, um das Reinigungsmedium zu dem zusätzlichen medizinischen Instrument zu fördern oder zu leiten,
und wobei die Adaptionsleiste (AL) ein zusätzliches Adaptersperrelement aufweist, welches beweglich ausgeformt ist, um das zusätzliche Aufnahmeelement (200) zum Aufstecken des zusätzlichen Steckadapters des medizinischen Instruments freizugeben, wenn das zusätzliche Adaptersperrelement in einer ersten Stellung ist und/oder das zusätzliche Aufnahmeelement (200) zu sperren, wenn das zusätzliche Adaptersperrelement in einer zweiten Stellung ist, wobei das zusätzliche Adaptersperrelement zumindest ein zusätzliches Sperrstück zum Sperren eines Durchflusses des Reinigungsmediums von dem Versorgungskanal (KS) die zusätzliche Medienleitung aufweist, das derart ausgebildet ist, um in der ersten Stellung außerhalb der zusätzlichen Medienleitung angeordnet zu sein und in der zweiten Stellung innerhalb der zusätzlichen Medienleitung angeordnet ist.

12. Reinigungsgerät (WD) zur Reinigung von zumindest einem medizinischen Instrument (DI) mit einer Adaptionsleiste (AL) gemäß den Ansprüchen 1 bis 11.
